Europäisches Patentamt

European Patent Office

Office européen des brevets

(11) Veröffentlichungsnummer: **0 192 947**
**A2**

## (12) EUROPÄISCHE PATENTANMELDUNG

(21) Anmeldenummer: 86100610.4

(22) Anmeldetag: 17.01.86

(51) Int. Cl.⁴: **A 61 M 25/00**

(30) Priorität: 26.02.85 DE 3506750

(43) Veröffentlichungstag der Anmeldung:
03.09.86 Patentblatt 86/36

(84) Benannte Vertragsstaaten:
AT BE CH DE FR GB IT LI LU NL SE

(71) Anmelder: INTERMEDICAT GMBH
Gerliswilstrasse 74
CH-6020 Emmenbrücke(CH)

(72) Erfinder: Herlitze, Gerhard
Binsdorfer Strasse 4
D-3507 Baunatal 7(DE)

(74) Vertreter: von Kreisler, Alek, Dipl.-Chem. et al,
Deichmannhaus am Hauptbahnhof
D-5000 Köln 1(DE)

(54) Verfahren zum Erzeugen von Farbmarkierungen auf einem Katheter-Führungsdraht.

(57) Zur Erzeugung ringförmiger Farbmarkierungen (12) an einem spiralförmigen Katheter-Führungsdraht (10) wird der Führungsdraht an den für die Farbmarkierungen (12) vorgesehenen Stellen selektiv auf eine Anlaßtemperatur im Bereich von 200°C bis 360°C erwärmt, so daß sich an diesen Stellen bleibende Anlaßverfärbungen des Stahls ergeben. Das Verfahren hat den Vorteil, daß die Farbmarkierungen nicht durch Materialauftrag oder Materialabtrag hergestellt werden und daß die Kontur des Führungsdrahtes (10) nicht verändert wird. Es kann auch kein am Führungsdraht haftendes Fremdmaterial in den Körper des Patienten gelangen.

Croydon Printing Company Ltd

0192947

Verfahren zum Erzeugen von Farbmarkierungen auf einem
Katheter-Führungsdraht

Die Erfindung betrifft ein Verfahren zum Erzeugen von
Farbmarkierungen auf einem Katheter-Führungsdraht aus
Stahl, sowie eine Vorrichtung zur Durchführung des Verfahrens und einen mit Farbmarkierungen versehenen
Katheter-Führungsdraht aus Stahl.

Es ist bekannt, zum Einführen eines Katheters in einen
menschlichen Körper einen metallischen Führungsdraht zu
verwenden, der den Katheter versteift und der nach dem
Verlegen des Katheters aus diesem herausgezogen wird.
Diese Einführungstechnik wird als Seldinger-Technik
bezeichnet und die entsprechenden Führungsdrähte werden
Seldinger-Führungsdrähte genannt. Damit der Führungsdraht die erforderliche Flexibilität erhält, um sich
dem Verlauf von Blutgefäßen anpassen zu können, besteht
er aus einer oder mehreren spiralförmig gewickelten
Adern. Der Führungsdraht kann auch eine Seele aus in
Längsrichtung verlaufenden Adern aufweisen.

Die bekannten Führungsdrähte sind mit Längenmarkierungen versehen, um feststellen zu können, wie groß die in den Körper eingeführte Länge des Markierungsdrahtes ist. Die Längenmarkierungen werden durch Bedrucken, Prägen oder mittels Laserstrahlen erzeugt. Diese Verfahren haben den Nachteil, daß die erzeugten Druckbilder erhaben oder beim Laserverfahren vertieft sind. Bei Anwendung von Druckfarbe setzt sich Druckfarbe zwischen den Spiralwindungen ab, wodurch die Spiralwindungen verklebt werden. Die Druckfarbe kann bei Verformung des Führungsdrahtes abplatzen. Wenn die Farbmarkierungen aus einer aufgeklebten Prägefolie besteht, haftet diese nur an der schmalen Umfangsfläche des Spiraldrahtes. Infolge der kleinen Haftfläche kann sich die Prägefolie leicht ablösen. Eine weitere Möglichkeit der Anbringung von Markierungen besteht darin, den Spiraldraht zu ätzen. Hierbei kann trotz mehrfachen Spülens nicht sichergestellt werden, daß nicht noch Reste des Ätzmittels zwischen den Spiralwindungen oder zwischen Spirale und Seele vorhanden sind.

Der Erfindung liegt die Aufgabe zugrunde, ein Verfahren der eingangs genannten Art anzugeben, das ohne Auftrg von Fremdmaterial und ohne Beeinflussung der Außenkontur des Führungsdrahtes anwendbar ist und bei dem sichergestellt ist, daß kein Fremdmaterial am Führungsdraht verbleibt.

Das erfindungsgemäße Verfahren ist gekennzeichnet durch selektive Erwärmung der für die Farbmarkierungen vorgesehen Stellen auf eine Temperatur, bei der sich eine bleibende Anlaßfarbe ergibt.

Der Erfindung liegt der Gedanke zugrunde, die Anlaßfarbe des Stahls des Führungsdrahtes für die Erzeugung von Farbmarkierungen auszunutzen. Durch Wärmeeinwirkung lassen sich Markierungsringe unterschiedlicher Farben aufbringen. Das Verfahren ist auf einfache Weise durchführbar. Es hat den Vorteil, daß die Kontur des Führungsdrahtes nicht verändert wird und daß weder ein Materialabtrag noch ein Fremdmaterialauftrag erfolgt. Die Markierungsringe werden durch Anlassen des Stahls bleibend gefärbt. Der Führungsdraht kann auf einfache Weise sterilisiert und steril gehalten werden und es besteht keine Gefahr, daß Fremdmaterialien in den Körper des Patienten eindringen.

Die Erwärmung erfolgt vorzugsweise auf eine Temperatur zwischen etwa 200°C und 300°C. Zur Erzeugung verschiedenfarbiger Markierungen können unterschiedliche Stellen auf verschiedene Temperaturen aufgeheizt werden.

Eine Vorrichtung zur Durchführung des erfindungsgemäßen Verfahrens weist eine ringförmige induktive Erwärmungsvorrichtung auf, die einen Abschnitt des Führungsdrahtes umgibt. Die Erwärmung erfolgt hierbei selektiv in demjenigen Abschnitt des Führungsdrahtes, der sich in der Erwärmungsvorrichtung befindet. Nachdem dieser Abschnitt auf die gewünschte Anlaßtemperatur erwärmt worden ist, wird der Führungsdraht weiterbewegt, so daß ein anderer Abschnitt in den Bereich der Erwärmungsvorrichtung gelangt. Es können auch mehrere Erwärmungsvorrichtungen hintereinander angeordnet sein, die gleichzeitig auf mehrere Abschnitte des Führungsdrahtes einwirken.

Ein nach dem erfindungsgemäßen Verfahren mit Farbmarkierungen versehener Führungsdraht zeichnet sich dadurch aus, daß die Farbmarkierungen aus Anlaßfarben des Stahls bestehen.

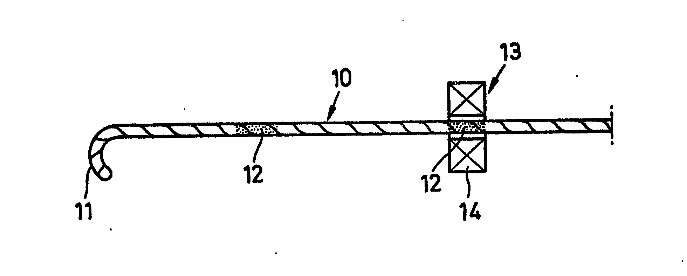

Im folgenden wird unter Bezugnahme auf die einzige Figur der Zeichnung ein Ausführungsbeispiel der Erfindung näher erläutert.

In der Zeichnung ist ein Katheter-Führungsdraht in Seitenansicht dargestellt, an dem Farbmarkierungen erzeugt werden.

Der Führungsdraht 10 besteht aus einem langgestreckten Strang aus mindestens einem spiralförmig gewickelten Draht, wobei die Drahtwindungen mit solcher Steigung gewickelt sind, daß benachbarte Drahtwindungen einander berühren. Der Führungsdraht enthält außerdem eine (nicht dargestellte) Drahtseele. Das vorderer Ende 11 des Führungsdrahtes 10 ist hakenförmig umgebogen, kann jedoch auch geradlinig verlaufen. Der Führungsdraht hat eine Stärke von z.B. 1 mm und eine Länge von z.B. 30 cm oder mehr.

Der Führungsdraht 10 ist mit mehreren mit gegenseitigen Abständen angeordneten Farbmarkierungen 12 versehen. Diese Farbmarkierungen sind als Ringe ausgebildet, die von jeder Stelle des Umfangs des Führungsdrahtes sichtbar sind. Die Farbmarkierungen haben beispielsweise eine Länge von 2 mm.

Zur Erzeugung der Farbmarkierungen dient eine induktive Erwärmungsvorrichtung 13, die beispielsweise aus einer ringförmigen Spule 14 besteht, durch die der Führungsdraht 10 hindurchgeschoben wird. Wenn sich eine Stelle, an der eine Farbmarkierung 12 angebracht werden soll, im Inneren der Spule 14 befindet, wird der Führungsdraht angehalten, damit diese Stelle induktiv erwärmt wird. Die Spule 14 weist zahlreiche Windungen auf, die eine Primärwicklung bilden, welche an eine Stromquelle angeschlossen wird. Die Drahtwindungen des Führungsdrahtes bilden die Sekundärwicklung. In diesen Sekundärwicklungen werden Ströme mit hoher Stromstärke erzeugt, wodurch eine selektive Erwärmung des in der Spule befindlichen Abschnitts des Führungsdrahtes erfolgt.

In der nachstehenden Tabelle I sind die sich bei normalen Stählen bei den angegebenen Temperaturen ergebenden Anlaßfarben aufgeführt:

TABELLE I

200°C - Weißgelb
220°C - Strohgelb
230°C - Golbgeld
240°C - Gelbbraun
250°C - Braunrot
260°C - Rot
270°C - Purpurrot
280°C - Violett
290°C - Dunkelblau
300°C - Kornblumenblau
320°C - Hellblau
340°C - Blaugrau
360°C - Grau.

Bei hochlegierten Stählen treten die angegebenen Anlaßfarben erst bei höheren Hitzegraden auf. Die Anlaßzeit beeinflußt die Anlaßfarben in dem Sinne, daß längeres Anlaßen bei niedriger Temperatur dieselbe Anlaßfarbe ergibt wie kürzeres Anlaßen bei höherer Temperatur.

Die induktive Erwärmungsvorrichtung 13 kann durch einen (nicht dargestellten) Temperaturfühler oder durch einen Strahlungssensor, der auf eine selektive Farbe der Farbmarkierung 12 reagiert, gesteuert sein, um die gewünschte Farbe einzustellen. Es besteht auch die Möglichkeit, die induktive Erwärmungsvorrichtung 13 ständig eingeschaltet zu lassen zu den Vorschub des Führungsdrahtes in Abhängigkeit von der Temperatur bzw. der erreichten Anlaßfarbe zu steueren.

## ANSPRÜCHE

1. Verfahren zum Erzeugen von Farbmarkierungen auf einem Katheter-Führungsdraht (10) aus Stahl, g e k e n n z e i c h n e t d u r c h, selektive Erwärmung der für die Farbmarkierungen (12) vorgesehenen Stellen auf eine Temperatur, bei der sich eine bleibende Anlaßfarbe ergibt.

2. Verfahren nach Anspruch 1, dadurch gekennzeichnet, daß die Erwärmung auf eine Temperatur zwischen etwa 200°C und 360°C erfolgt.

3. Verfahren nach Anspruch 1 oder 2, dadurch gekennzeichnet, daß zur Erzeugung verschiedenfarbiger Markierungen unterschiedliche Stellen auf unterschiedliche Temperaturen aufgeheizt werden.

4. Vorrichtung zur Durchführung des Verfahrens nach einem der Ansprüche 1 bis 3, dadurch gekennzeichnet, daß eine ringförmige induktive Erwärmungsvorrichtung (13) vorgesehen ist, die einen Abschnitt des Führungsdrahtes (10) umgibt.

5. Katheter-Führungsdraht aus Stahl, mit Farbmarkierungen, dadurch gekennzeichnet, daß die Farbmarkierungen (12) aus Anlaßfarben des Stahls bestehen.